# EUROPÄISCHE PATENTSCHRIFT

(11) **EP 4 041 365 B1**
(45) Veröffentlichungstag und Bekanntmachung des Hinweises auf die Patenterteilung: **02.07.2025**
(21) Anmeldenummer: 20810927.2
(22) Anmeldetag: 19.11.2020
(51) Int. Cl.: A61M 39/10, A61M 39/26

(54) **KUPPLUNGSSYSTEM FÜR EIN GESCHLOSSENES FLUIDTRANSFERSYSTEM**
COUPLING SYSTEM FOR A CLOSED FLUID TRANSFERSYSTEM
SYSTEME D'ACCOUPLEMENT POUR UN SYSTEM DE TRANSFERT DE FLUID FERME

(30) Priorität: 21.11.2019 DE 102019217987
(43) Veröffentlichungstag der Anmeldung: 17.08.2022
(73) Patentinhaber: B. Braun Melsungen AG, 34212 Melsungen (DE)
(72) Erfinder: BERG, Karl Martin, 34212 Melsungen (DE); FISCHER, Nathanael, 36251 Bad Hersfeld (DE); KOPP, Florin, 26419 Schortens (DE); SEIDEL, Gerrit, 34121 Kassel (DE)
(74) Vertreter: Maiwald GmbH
(86) Internationale Anmeldenummer: PCT/EP2020/082624
(87) Internationale Veröffentlichungsnummer: WO 2021/099438

(56) Entgegenhaltungen:
- US-A- 5 492 147
- US-A1- 2005 087 715
- US-A1- 2019 184 152

## Beschreibung

Verschiedene Arzneimittel können bei unsachgemäßem Kontakt eine gesundheitsgefährdende Wirkung aufweisen. Beispielsweise schädigen CMR-Arzneimittel (Cancorogen-Mutagen-Reprotoxic-Arzneimittel), wie sie zur Krebstherapie eingesetzt werden, bei der therapeutischen Anwendung vor allem wachstumsintensive Tumorzellen. Viele dieser Arzneimittel haben bedingt durch ihren Wirkmechanismus selbst krebserzeugende Eigenschaften. Um den Kontakt nicht in Therapie befindlicher Personen mit CMR-Arzneimitteln zu verhindern, werden bei der Herstellung und Verabreichung applikationsfertiger Zubereitungen vermehrt geschlossene Fluidtransfersysteme, sogenannte "Closed System Transfer Devices" (CSTD) eingesetzt. Wichtiger Bestandteil dieser geschlossenen Fluidtransfersystem sind Kupplungssysteme, die den sicheren Transfer von gesundheitsgefährdenden Substanzen, wie CMR-Arzneimitteln, ermöglichen und nach Trennen der Verbindung trocken abschließen, um so die Umgebung vor einer Kontamination, beispielsweise durch Leckagen oder Tropfenbildung auf der Oberfläche der Kupplungspartner nach Trennen der Verbindung, zu schützen.

Kupplungssysteme dieser Art werden im Allgemeinen mit den Begriffen "Dry Connection", "Automatic Self-Sealing Technology" oder auch "Closed Connection" in Verbindung gebracht und sind wesentlicher Bestandteil zur Realisierung von geschlossenen Fluidtransfersystemen, die u.a. für die Verstellung und Verabreichung applikationsfertiger CMR-Arzneimittel immer bedeutsamer werden.

Bei einem bekannten Kupplungssystem basiert beispielsweise die Innenseite der Kupplung auf einem Luer-Lock, wodurch sich ein kleiner Flussspalt mit nur sehr geringem Durchfluss ergibt. Andere Kupplungssysteme können aufgrund schlecht übereinanderliegender Geometrien der Flussspalte in den verwendeten Elastomeren höhere Fluidrückstände auf den Elastomeroberflächen aufweisen. Zudem ist die Desinfizierbarkeit der Kupplungsoberflächen üblicher Kupplungssysteme darüber hinaus erschwert, da zumindest einer der Kupplungspartner eine rückversetzte und somit schlecht zugängliche Kupplungsoberfläche aufweist.

In diesem Zusammenhang offenbart ergänzend die US 2005/0087715 A1 einen männlichen Luer-Anschluss, der an einen standardmäßigen weiblichen Luer-Anschluss angeschlossen wird, um einen Strömungskanal für medizinische Flüssigkeiten zwischen den beiden Anschlüssen zu öffnen. Der Stecker hat zwei interne Ventile und eine vakuumerzeugende Struktur.

Die US 5492147 A betrifft ein Kupplungssystem zum Zusammenfügen gegenüberliegender Durchgänge und umfasst ein männliches Element und ein weibliches Element, von denen jedes eine elastisch verformbare Gummiklappe mit Fadenkreuzschlitzen umfasst, die so positioniert sind, dass sie ineinander eingreifen, wenn das männliche und das weibliche Element axial in ihre gekoppelte Position bewegt werden.

Die US 2019/0184152 A1 betrifft einen männlichen Verbinder, der mit einem weiblichen Verbinder verbindbar ist und ein röhrenförmiges Strömungspfadelement, das eine Öffnung an einem Endabschnitt aufweist, einen Ventilkörper, der die Öffnung schließt, einen Gehäusehauptkörper und einen beweglichen Körper, der sich in Bezug auf den Gehäusehauptkörper bewegt und den Ventilkörper verformt, um einen Modus zwischen einem ersten Modus, in dem die Öffnung durch den Ventilkörper geschlossen ist, und einem zweiten Modus, in dem die Öffnung von dem geöffnet ist, zu ändern, umfasst.

In Anbetracht der mit dem Stand der Technik verbundenen Nachteile ist es Aufgabe der vorliegenden Erfindung, ein Kupplungssystem für ein geschlossenes Fluidtransfersystem bereitzustellen, das den sicheren Transfer eines Fluids in einem geschlossenen Fluidtransfersystem in einem konnektierten Zustand verbessert und im dekonnektierten Zustand eine Kontamination der Umgebung minimieren und möglichst verhindert.

Die erfindungsgemäße Aufgabe wird durch ein Kupplungssystem für ein geschlossenes Fluidtransfersystem nach Anspruch 1 gelöst. Weitere vorteilhafte Ausgestaltungen der Erfindung ergeben sich aus den Unteransprüchen.

Erfindungsgemäß umfasst das Kupplungssystem für ein geschlossenes Fluidtransfersystem ein Kupplungselement. Das Kupplungselement umfasst ein Kupplungselementgehäuse mit einem Fluidanschluss und einer Kupplungsseite, wobei das Kupplungselementgehäuse eine sich von dem Fluidanschluss in Richtung der Kupplungsseite erstreckende Längsachse aufweist, einen Fluidkanal, der sich in Richtung der Längsachse vom Fluidanschluss in das Kupplungselementgehäuse erstreckt, ein Kupplungselementdichtelement, das eine der Kupplungsseite zugewandte Fluidkanalöffnung des Fluidkanals überdeckt, wobei das Kupplungselementdichtelement zumindest eine reversibel öffenbare und schließbare Kupplungselementdichtelementöffnung im Bereich der Überdeckung der der Kupplungsseite zugewandten Fluidkanalöffnung aufweist und zumindest ein sich an die Kupplungselementdichtelementöffnung anschließender und diese umfassender Bereich, insbesondere das gesamte Kupplungselementdichtelement, aus einem elastischen Material ausgebildet ist, und zumindest ein Aktivierungselement, das an dem Kupplungselementdichtelement angreift und in Richtung der Längsachse im Kupplungselementgehäuse zwischen einer Position mit maximalem Abstand zum Fluidanschluss und einer Position mit minimalem Abstand zum Fluidanschluss bewegbar ist, wobei das Aktivierungselement derart konfiguriert ist, dass die Kupplungselementdichtelementöffnung des Kupplungselementdichtelements in der Position des Aktivierungselements mit maximalem Abstand zum Fluidanschluss geschlossen ist, und dass die Kupplungselementdichtelementöffnung in der Position des Aktivierungselements mit minimalem Abstand zu Fluidanschlusses geöffnet ist.

Die Position des Aktivierungselements mit maximalen Abstand zum Fluidanschluss entspricht einer Position des Aktivierungselements im dekonnektierten Zustand, ohne dass ein Gegenkupplungselement am Kupplungselement angreift. Die Position des Aktivierungselements mit minimalem Abstand zum Fluidanschluss korrespondiert zu einer Position des Aktivierungselements im konnektierten Zustand, im dem das Gegenkupplungselement zum sicheren Fluidtransfer mit dem Kupplungselement konnektiert ist. Alternativ kann die Position des Aktivierungselements mit minimalem Abstand zum Fluidanschluss auch eine Position mit geringerem Abstand zum Fluidanschluss als der Abstand im konnektierten Zustand sein. Im Sinne eines sicheren Öffnens der Kupplungselementdichtelementöffnung durch das Aktivierungselement ist jedoch die Position des Aktivierungselements mit minimalem Abstand, die der Position des Aktivierungselements im konnektierten Zustand entspricht, ausreichend.

Durch die Bewegung des Aktivierungselements zwischen den vorgenannten Positionen, also den Positionen mit minimalem und maximalem Abstand, erfolgt eine Relativbewegung zwischen dem Aktivierungselement und Fluidkanal. Da das Aktivierungselement zudem am Kupplungselementdichtelement angreift, wird zumindest der Bereich des Kupplungselementdichtelements im Angriffspunkt des Aktivierungselements relativ mitbewegt. Da wiederum das Kupplungselementdichtelement die der Kupplungsseite zugewandte Fluidkanalöffnung des Fluidkanals überdeckt und die zumindest eine reversibel öffenbare und schließbare Kupplungselementdichtelementöffnung in diesem Bereich der Überdeckung aufweist, wirkt über die durch das Aktivierungselement initiierte Relativbewegung eine Zugkraft auf den sich an die Kupplungselementdichtelementöffnung anschließenden und diese umfassenden Bereich aus elastischem Material. Infolge der Verformung des Bereichs aus elastischem Material wird die Kupplungselementdichtelementöffnung mit zunehmender Relativbewegung und somit zunehmender Zugkraft geöffnet. Im Sinne des reversiblen Öffnens und Schließens der Kupplungselementdichtelementöffnung wird bevorzugt die Position des Aktivierungselements mit minimalem Abstand zum Fluidanschluss auf eine Position und demnach auf eine maximale Bewegungsstrecke begrenzt, bei der die resultierende Zugkraft weder die Festigkeit eines der Zugkraft ausgesetzten Materials des Kupplungselementdichtelement übersteigt noch in eine plastischen Verformung eines der Zugkraft ausgesetzten Materials des Kupplungselementdichtelement bewirkt. Gemäß den vorstehenden Ausführungen lässt sich somit über die Bewegung des Aktivierungselements relativ zum Fluidkanal in Richtung der Längsachse L1 das reversible Öffnen und Schließen der Kupplungselementdichtelementöffnung in einfacher Weise realisieren.

Da das Aktivierungselement bei Bewegung in Richtung des Fluidanschlusses das Kupplungselementdichtelement spannt, also quasi gegen eine Federkraft des Kupplungselementdichtelements arbeitet, ist das Kupplungselementdichtelement bestrebt, mit Wegfall der durch das Aktivierungselement aufgebracht Kraft, wieder in seinen Ausgangszustand zurückzukehren. Wird das Aktivierungselement also nicht beabsichtigt aus einer Position mit maximalem Abstand zum Fluidanschluss bewegt oder in einer Position gehalten, die nicht der Position mit maximalem Abstand zum Fluidanschluss entspricht, wird es wiederum durch das Kupplungselementdichtelement in diese Position zurückgeführt und die Kupplungselementdichtelementöffnung automatisch geschlossen.

Zudem umfasst das Kupplungssystem ein Gegenkupplungselement, umfassend ein Gegenkupplungselementgehäuse mit einem Gegenkupplungselementfluidanschluss und einer Gegenkupplungsseite, wobei das Gegenkupplungselementgehäuse eine sich von dem Gegenkupplungselementfluidanschluss in Richtung der Gegenkupplungsseite erstreckende Gegenkupplungselementlängsachse aufweist, und ein Gegenkupplungselementdichtelement, das in dem Gegenkupplungselementgehäuse angeordnet ist und zusammen mit dem Gegenkupplungselementgehäuse zumindest einen Teil einer gegenkupplungsseitigen Stirnfläche des Gegenkupplungselements ausbildet, wobei das Gegenkupplungselementdichtelement im Bereich der gegenkupplungsseitigen Stirnfläche eine reversibel öffenbare und schließbare Gegenkupplungselementdichtelementöffnung aufweist und zumindest ein sich an die Gegenkupplungselementdichtelementöffnung anschließender und diese umfassender Bereich, insbesondere das gesamte Gegenkupplungselementdichtelement, aus einem elastischen Material ausgebildet ist.

Durch die zumindest teilweise elastische Ausbildung des Gegenkupplungsdichtelements lässt sich dieses vergleichbar zum Kupplungselementdichtelement durch Kraftbeaufschlagung in einfacher Weise reversibel öffnen und schließen. In einem dekonnektierten Zustand, der einem Zustand entspricht, in dem keine Kraftbeaufschlagung erfolgt, ist die Gegenkupplungselementdichtelementöffnung geschlossen. Wird nun die zur Gegenkupplungsseite weisende Stirnseite, also der Bereich der Gegenkupplungselementdichtelementöffnung mit einer Kraft in Richtung des Gegenkupplungselementfluidanschlusses beaufschlagt, wird zumindest der Bereich der Gegenkupplungselementdichtelementöffnung in Richtung des Gegenkupplungselementfluidanschlusses eingedrückt und somit die Gegenkupplungselementdichtelementöffnung geöffnet. Wird eine solche Kraftbeaufschlagung zurückgenommen, formt sich der Bereich der Gegenkupplungselementdichtelementöffnung durch seine elastische Ausbildung wider in eine Ausgangsform zurück, wodurch die Gegenkupplungselementdichtelementöffnung wieder geschlossen wird.

Die reversibel öffenbare und schließbare Gegenkupplungselementdichtelementöffnung wird in einem konnektierten Zustand durch das Kupplungselementdichtelement geöffnet.

Hierzu ist das Kupplungselementdichtelement im Kontaktbereich mit der ihm zugewandten Oberfläche des Gegenkupplungselementdichtelements zur Öffnung der Gegenkupplungselementdichtelementöffnung weniger leicht elastisch verformbar als der korrespondierende Kontaktbereich des Gegenkupplungselementdichtelements. Mit andern Worten bedingt das Kupplungselementdichtelement die Verformung des Gegenkupplungselementdichtelements zur Öffnung der Gegenkupplungselementdichtelementöffnung und wird nicht selbst durch das Gegenkupplungselementdichtelement verformt. Alternativ oder ergänzend erfolgt die Öffnung der Gegenkupplungselementdichtelementöffnung mittelbar über das der Kupplungsseite zugewandte Ende des Fluidkanals, gegen den sich die der Kupplungsseite zugewandte Fläche des Gegenkupplungselementdichtelements in diesem Bereich abstützt.

Durch die Öffnung der reversibel öffenbaren und schließbaren Gegenkupplungselementdichtelementöffnung durch das Kupplungselementdichtelement im konnektierten Zustand werden die jeweiligen Dichtelementflächen aufeinandergedrückt, so das eine trockene Abdichtung und somit trockene Oberflächen erreicht werden können. Durch die elastische Verformbarkeit erfolgt eine Öffnung der reversibel öffenbaren und schließbaren Gegenkupplungselementdichtelementöffnung also erst mit Kontaktierung durch das Kupplungselementdichtelement. Würde die reversibel öffenbare und schließbare Gegenkupplungselementdichtelementöffnung im Gegensatz dazu beispielsweise derart geöffnet, dass ein Bereich ihrer im dekonnektierten Zustand nach außen weisenden Oberfläche bei einem Flüssigkeitsaustausch von dem jeweiligen Fluid umströmt werden, besteht das Risiko, dass Patienten und/oder medizinisches Personal nach einer Dekonnektierung mit diesen verunreinigten Flächen in Kontakt kommen.

In einer Ausgestaltung umgibt das Kupplungselementdichtelement ausgehend von dem Bereich der Überdeckung der der Kupplungsseite zugewandten Fluidkanalöffnung den Fluidkanal in Richtung des Fluidanschlusses zumindest über einen vorbestimmten Abschnitt in Bezug auf die Längsachse radial und weist einen in Bezug auf die Längsachse radial nach außen weisenden Vorsprung, insbesondere an einem dem Fluidanschluss zugewandten Ende des Kupplungselementdichtelements, auf, an dem ein dem Fluidanschluss zugewandtes Ende oder ein in Bezug auf die Längsachse radial nach innen weisender Vorsprung des Aktivierungselements angreift.

Über eine solche Konfiguration wird nicht nur die der Kupplungsseite zugewandte Öffnung des Fluidkanals über das Kupplungselementdichtelement abgedichtet, sondern auch sich hieran anschließender in Bezug auf die Längsachse axialer Abschnitt des Fluidkanals. Neben der Möglichkeit, hierdurch auch Bereiche des überdeckten axialen Abschnitts zu überdecken, erhöht sich gleichfalls die Sicherheit der fluiddichten Überdeckung der der Kupplungsseite zugewandten Öffnung des Fluidkanals bzw. verringern sich die Toleranzanforderungen gegenüber einer rein stirnseitigen Überdeckung. Sofern das gesamte Kupplungselementdichtelement oder zumindest der den Fluidkanal umgebende Teil des Kupplungselementdichtelements elastisch ist, kann durch leichte Aufdehnung des Kupplungselementdichtelements bei der Anordnung um den Fluidkanal eine ausreichende Haltekraft vorgesehen werden.

Der in Bezug auf die Längsachse radial nach außen weisende Vorsprung des Kupplungselementdichtelements, an dem ein dem Fluidanschluss zugewandtes Ende oder ein in Bezug auf die Längsachse radial nach innen weisender Vorsprung des Aktivierungselements angreift, ermöglicht eine Dehnung des den Fluidkanal in Richtung der Längsachse umgebenden Abschnitts des Kupplungselementdichtelements, wenn das Aktivierungselement entlang der Längsachse in Richtung des Fluidanschlusses bewegt wird. Über diese Dehnung wird eine Zugkraft aufgebracht, die die Kupplungselementdichtelementöffnung reversibel öffnet.

Wenn der in Bezug auf die Längsachse radial nach außen weisende Vorsprung des Kupplungselementdichtelements an einem dem Fluidanschluss zugewandten Ende des Kupplungselementdichtelements vorgesehen ist, kann die die Position des Aktivierungselements mit minimalem Abstand zum Fluidanschluss, die dann einem konnektierten Zustand entspricht, eine Position sein, in der der radial nach außen weisende Vorsprung des Kupplungselementdichtelements zwischen dem dem Fluidanschluss zugewandten Ende des Aktivierungselements und einem diesem Ende gegenüberliegenden Abschnitt des Kupplungselementgehäuses 10 eingeklemmt wird. Das Kupplungselementdichtelement wird demnach im konnektierten Zustand sicher gehalten, wodurch die Öffnung der Kupplungselementdichtelementöffnung gewährleistet wird.

Der radial nach außen weisende Vorsprung des Kupplungselementdichtelements kann durchgängig ringförmig um den Fluidkanal ausgebildet sein oder auch nur abschnittsweise.

In einer Weiterbildung umfasst der Fluidkanal zwei durch eine Trennwand voneinander getrennte Fluidkanalabschnitte, die jeweils eine seitliche Fluidkanalabschnittsöffnung aufweisen, und das Kupplungselementdichtelement weist eine Fluidkammer auf, die sich zwischen einem kupplungsseitigen Kupplungselementdichtelementabschnitt und einem fluidanschlussseitigen Kupplungselementdichtelementabschnitt in Bezug auf die Längsachse radial um den Fluidkanal erstreckt, wobei der fluidanschlussseitige Kupplungselementdichtelementabschnitt in einem Zustand, in dem sich das Aktivierungselement in der Position mit maximalem Abstand zum Fluidanschluss befindet, zumindest die Fluidkanalabschnittsöffnung des fluidanschlussseitigen Fluidkanalabschnitts abdichtet und sich die Fluidkammer in einem Zustand, in dem sich das Aktivierungselement in der Position mit minimalen Abstand zum Fluidanschluss befindet, über die seitliche Fluidkanalabschnittsöffnung des kupplungsseitenseitigen Fluidkanalabschnitts und die seitliche Fluidkanalabschnittsöffnung des fluidanschlussseitigen Fluidkanalabschnitts erstreckt, um über die Fluidkammer eine fluide Verbindung zwischen dem kupplungsseitenseitigen Fluidkanalabschnitt und dem fluidanschlussseitigen Fluidkanalabschnitt auszubilden.

Mit anderen Worten wird der Fluidkanal über die Trennwand in zwei Bereiche unterteilt, wobei über die seitlichen Fluidkanalabschnittsöffnungen, das heißt, über einen Außenflächenabschnitt des Fluidkanals ein Fluidaustausch erfolgen kann. Wenn sich das Aktivierungselement in einer Position mit maximalem Abstand zum Fluidanschluss befindet, wird jedoch gemäß der vorliegenden Weiterbildung die fluidanschlussseitige Fluidkanalabschnittsöffnung über den fluidanschlussseitigen Kupplungselementdichtelementabschnitt abgedichtet, so dass kein Fluidaustausch erfolgen kann. Die der Kupplungsseite zugewandte Öffnung des kupplungsseitigen Fluidkanalabschnitts ist in diesem Zustand durch die geschlossene Kupplungselementdichtelementöffnung abgedichtet. Die Sicherheit gegen ein Austreten eines Fluids ist somit erhöht. Zudem weist das Kupplungselementdichtelement eine den Fluidkanal in Bezug auf die Längsachse radial umgebende Fluidkammer auf, die die kupplungsseitige Fluidkanalabschnittsöffnung umgibt. Wird nun das Aktivierungselement aus einer Position mit maximalem Abstand zu Fluidanschluss in eine Position mit minimalem Abstand zum Fluidanschluss bzw. in einen konnektierten Zustand bewegt, wird die Fluidkammer in Richtung der Längsachse vergrößert. Bevorzugt ist zumindest der die Fluidkammer umfassende Abschnitt des Kupplungselementdichtelements elastisch ausgebildet, so dass die Vergrößerung in Richtung der Längsachse einer Dehnung der Fluidkammer in dieser Richtung entspricht. Durch die Vergrößerung der Fluidkammer erstreckt sich diese in einer Position des Aktivierungselements mit minimalem Abstand zum Fluidanschluss bzw. in einem konnektierten Zustand zumindest abschnittsweise, insbesondere vollständig, sowohl über die kupplungsseitige als auch fluidanschlussseitige Fluidkanalabschnittsöffnung, so dass über die Fluidkammer ein Fluidaustausch zwischen dem kupplungsseitigen und fluidanschlussseitigen Fluidkanalabschnitt stattfinden kann. Gleichzeitig ist in dieser Position des Aktivierungselements die Kupplungselementdichtelementöffnung geöffnet, um den weiteren Fluidaustausch mit einem später noch beschriebenen Gegenkupplungselement zu ermöglichen. Somit kann die Öffnung der fluidanschlussseitigen Fluidkanalabschnittsöffnung und der Kupplungselementdichtelementöffnung über die alleinige Bewegung des Aktivierungselements erfolgen.

In einer Ausgestaltung weist der in Bezug auf die Längsachse nach außen weisende Vorsprung des Kupplungselementdichtelements einen Befestigungsabschnitt auf, der im Kupplungselementgehäuse befestigt ist.

Somit wird das Kupplungselementdichtelement sicher im Kupplungselementgehäuse gehalten, so dass das Risiko einer unbeabsichtigten Verschiebung und damit möglicherweise verbundenen Öffnung der Kupplungselementdichtelementöffnung verringert wird.

Insbesondere umfasst das Kupplungselementgehäuse entlang der Längsachse einen kupplungsseitenseitigen Gehäuseabschnitt und einen fluidanschlussseitigen Gehäuseabschnitt umfasst, zwischen denen der Befestigungsabschnitt des Kupplungselementdichtelements gehalten wird.

Beispielsweise kann der zuvor beschriebene radiale Vorsprung des Kupplungselementdichtelements oder ein weiterer sich daran radial nach außen anschließender Abschnitt für eine solche Befestigung zwischen den Gehäuseabschnitten genutzt werden.

In einer Ausgestaltung ist das Aktivierungselement ringförmig ausgebildet, wobei insbesondere der Ring des Aktivierungselements am zum Fluidanschluss weisenden Ende des Kupplungselementdichtelements angreift.

Die ringförmige Ausgestaltung stellt sicher, dass das Aktivierungselement in jeder Einsatzposition am Kupplungselementdichtelement angreift, auch wenn die zum Angriff durch das Aktivierungselement vorgesehenen Abschnitte des Kupplungselementdichtelements nur abschnittsweise vorgesehen sind. Zudem kann über eine ringförmige Ausgestaltung, zumindest im Bereich des der Kupplungsseite zugewandten Ende des Aktivierungselements, der zur Aufnahme und Bewegung des Aktivierungselements zwischen dem Kupplungselementgehäuse und dem Kupplungselementdichtelement vorgesehene Abstand im dekonnektierten Zustand bzw. in der Position des Aktivierungselements mit maximalem Abstand zum Fluidanschluss durch das Aktivierungselement geschlossen werden. In diesem Fall entspricht die Wandstärke des ringförmigen Bereichs dem zwischen dem Kupplungselementgehäuse und dem Kupplungselementdichtelement an der Kupplungsseite gebildeten Abstand.

Alternativ kann das Aktivierungselement zumindest im Bereich seines dem Fluidanschluss zugewandten Endes auch nur abschnittsweise ausgebildet sein, also beispielsweise nur jeweils in Richtung des Fluidanschlusses weisende Aktivierungselementabschnitte umfassen. In einem solchen Fall kann sichergestellt werden, dass die Kupplungselementdichtelementöffnung nur in bestimmten Einsatzpositionen des Aktivierungselements geöffnet werden kann. In Bezug auf das Aktivierungselement sei zudem angemerkt, dass dieses insgesamt ringförmig ausgebildet sein kann, aber den Fluidkanal nicht zwingend vollumfänglich umschließen muss. Ebenso ist es möglich, das Aktvierungselement nur über einen begrenzten in Bezug auf die Längsachse tangentialen Abschnitt oder mehrere entlang des Umfangs des Fluidkanals bzw. des Kupplungselementdichtelements verteilte Aktivierungselemente vorzusehen.

In einer Weiterbildung weist das Kupplungselementgehäuse auf der Kupplungsseite zumindest eine Haltestruktur, insbesondere eine in Bezug auf die Längsachse auf der Kupplungsseite axial über das Kupplungselementdichtelement vorstehende Haltestruktur, auf, über die ein Gegenkupplungselement in einem konnektierten Zustand gehalten werden kann.

Da zumindest ein Teil des Kupplungselementdichtelements elastisch ausgebildet ist und das Aktivierungselement bei Bewegung aus einer Position mit maximalem Abstand zum Fluidanschluss, in der zumindest der elastische Abschnitt nicht in Bezug auf die Bewegungsrichtung des Aktvierungselements vorgespannt ist, gegen eine Federkraft des Kupplungselementdichtelements arbeitet, ermöglicht die Haltestruktur die Beibehaltung des konnektierten Zustands, ohne dass weiterhin eine aktive Haltekraft, beispielsweise über einen Anwender, aufgebracht werden muss.

Insbesondere wird die Haltestruktur über zumindest zwei Haltearme mit Schnapphaken oder ringförmig ausgebildet.

Sofern lediglich abschnittsweise Haltearme vorgesehen sind, wird die Zugänglichkeit zu beispielsweise desinfizierenden Flächen, wie der kupplungsseitigen Stirnfläche des Kupplungselementdichtelements, verbessert. Die zumindest zwei Haltearme sind so angeordnet, dass ein Verkippen eines hierüber zu haltenden Gegenkupplungselements weitestgehend vermieden wird. Beispielsweise sind zwei Haltearme im Wesentlichen gegenüberliegend angeordnet oder drei Haltearme in einem Abstand von im Wesentlichen 120° zueinander. Eine ringförmige Ausbildung der Haltestruktur kann alternativ eine weitere Abdichtung zwischen dem Kupplungselement und einem hierzu korrespondierenden Gegenkupplungselement ermöglichen.

Die Verwendung von Schnapphaken zum Eingriff in ein Gegenkupplungselement ermöglicht die Befestigung des Gegenkupplungselements in einer vorbestimmten Position. Alternativ kann die Haltestruktur Gewindeabschnitte umfassen, um hierüber einen Toleranzbereich überbrücken oder anderweitige Positionsanpassungen vornehmen zu können.

In einer Ausgestaltung kann ein gegenkupplungsseitiges Ende des Gegenkupplungselementgehäuses an dem zumindest einen Aktivierungselement des Kupplungselements angreifen und ist mit diesem zusammen in Richtung des Fluidanschlusses des Kupplungselements in das Kupplungselementgehäuse bewegbar.

Das gegenkupplungsseitige Ende des Gegenkupplungselementgehäuses ist hierzu insbesondere ringförmig ausgebildet, kann aber auch nur abschnittsweise ausgebildet sein. Zudem weist das gegenkupplungsseitige Ende des Gegenkupplungselementgehäuses in Bezug auf die Gegenkupplungselementlängsachse eine radiale Dicke auf, die so konfiguriert ist, dass sie zwischen dem Kupplungselementgehäuse und dem Kupplungselementdichtelement in das Kupplungselementgehäuse bewegbar ist. Insbesondere entspricht die radiale Dicke im Wesentlichen der radialen Dicke des Aktivierungselements.

Insbesondere ist das gegenkupplungsseitige Ende des Gegenkupplungselementgehäuses als in Bezug auf die Gegenkupplungselementlängsachse axialer Vorsprung ausgebildet und bildet mit einem sich daran anschließenden Gehäuseabschnitt des Gegenkupplungselementgehäuses einen Schulterabschnitt.

Über den Schulterabschnitt kann Zusammenwirken mit dem Kupplungselementgehäuse ein Anschlag gebildet werden, dessen Erreichen einem konnektierten Zustand entspricht. Über die Bewegung des gegenkupplungsseitige Ende des Gegenkupplungselementgehäuses bis zum Erreichen des Anschlags in Richtung des Fluidanschlusses in das Kupplungselementgehäuse wird demnach wird das Aktivierungselement gleichsam in den konnektierten Zustand, insbesondere in die Position mit minimalem Abstand zum Fluidanschluss bewegt.

In einer Ausgestaltung weist das Gegenkupplungselementgehäuse an einer sich in Bezug auf die Gegenkupplungselementlängsachse axial erstreckenden Außenfläche eine Gegenkupplungselementhaltestruktur auf, über den das Gegenkupplungselement in einem konnektierten Zustand mit dem Kupplungselement gehalten werden kann.

Wie bereits zum Kupplungselement in Bezug auf das Aktivierungselement ausgeführt, kann das Gegenkupplungselement vergleichbar ohne Anwendung einer weiteren aktiven Haltekraft im konnektierten Zustand gehalten werden. Ohne weitere aktive Haltkraft oder die entsprechende Haltestruktur bzw. Gegenkupplungselementhaltestruktur würden sich das Gegenkupplungselement und das Kupplungselement andernfalls über das Rückformungsbestreben der elastisch ausgebildeten Bereich des Kupplungselementdichtelements und des Gegenkupplungselementdichtelements auseinanderdrücken.

In einer weiteren Ausgestaltung umfasst das Gegenkupplungselementgehäuse einen Gegenkupplungselementfluidkanal mit zumindest einer seitlichen Gegenkupplungselementfluidkanalöffnung, der sich in Richtung der Gegenkupplungselementlängsachse vom Gegenkupplungselementfluidanschluss in Richtung der Gegenkupplungsseite im Gegenkupplungselementgehäuse erstreckt, und das Gegenkupplungselementdichtelement dichtet in einem dekonnektierten Zustand die seitliche Gegenkupplungselementfluidkanalöffnung ab, wobei das Gegenkupplungselementdichtelement zwischen der Abdichtung und der Gegenkupplungselementdichtelementöffnung eine Gegenkupplungselementdichtelementfluidkammer aufweist, die in Richtung der Gegenkupplungselementlängsachse relativ zum Gegenkupplungselementfluidkanal bewegbar ist, um im konnektierten Zustand über die seitliche Gegenkupplungselementfluidkanalöffnung eine fluide Verbindung zum Inneren des Gegenkupplungselementfluidkanals auszubilden.

Da die Gegenkupplungselementdichtelementöffnung durch leichtes Eindrücken unbeabsichtigt geöffnet werden könnte, wird über die Abdichtung der Gegenkupplungselementfluidkanalöffnung über das Gegenkupplungselementdichtelement der unbeabsichtigte Austritt eines im Fluidkanal eventuell befindlichen Fluids aus dem Gegenkupplungselementfluidkanal verhindert. Um eine fluide Verbindung zwischen dem Gegenkupplungselementfluidkanal und der Gegenkupplungselementdichtelementfluidkammer vorzusehen, kann die Gegenkupplungselementdichtelementfluidkammer in Richtung des Gegenkupplungselementfluidanschlusses entlang der Gegenkupplungselementlängsachse relativ zum Gegenkupplungselementfluidkanal bewegt werden. Dies kann durch eine Verschieben des Gegenkupplungselementdichtelements in Richtung des Gegenkupplungselementfluidanschlusses oder ein Komprimieren zumindest eines sich in Richtung des Gegenkupplungselementfluidanschlusses an die Gegenkupplungselementdichtelementfluidkammer anschließenden Abschnitts des Gegenkupplungselementdichtelements, der insbesondere entsprechend aus elastischen Material ausgebildet ist, erfolgen. Sofern eine Verschiebung vorgesehen ist, stützt sich das Gegenkupplungselementdichtelement an einem dem Gegenkupplungselementfluidanschluss zugewandten Ende des Gegenkupplungselementdichtelements auf einem Federelement ab, dass bei Verschiebung entgegen seiner Federkraft komprimiert wird, um bei Entlastung das Gegenkupplungselementdichtelement wieder in seine Ausgangsposition im Sinne eines dekonnektierten Zustands zurückzubewegen. Sofern das Gegenkupplungselementdichtelement selbst oder zumindest eine Abschnitt des Gegenkupplungselementdichtelements komprimiert wird, stützt sich das Gegenkupplungselementdichtelement insbesondere an einem dem Gegenkupplungselementfluidanschluss zugewandten Ende des Gegenkupplungselementdichtelements an einem diesem Ende zugewandten Gegenkupplungselementgehäuseabschnitt ab, um lediglich komprimiert und nicht verschoben zu werden, so dass das Gegenkupplungselementdichtelement bei Dekomprimierung wieder seinen Ausgangszustand erreicht. Die beiden Varianten können auch miteinander kombiniert werden, um beispielsweise größere Bewegungsstrecken der Fluidkammer zu realisieren.

Die Erfindung betrifft zudem ein Kupplungssystem für ein geschlossenes Fluidtransfersystem, umfassend zumindest ein erfindungsgemäßes Kupplungselement sowie zumindest ein erfindungsgemäßes Gegenkupplungselement, wobei das Gegenkupplungselement derart konfiguriert ist, dass es das Aktivierungselement bei einer Kupplung in die Position mit minimalem Abstand zum Fluidanschluss bewegt, wobei eine durch die Bewegung des Aktivierungselements bedingte Öffnung der Kupplungselementdichtelementöffnung erst erfolgt, wenn die Kupplungselementdichtelementöffnung über das Gegenkupplungselement nach außen abgedichtet ist.

Eine entsprechende Abdichtung wird insbesondere dann erreicht, wenn die einander zugewandten Oberflächen des Kupplungselementdichtelements und des Gegenkupplungselementdichtelements mit einer vorbestimmten Druckkraft gegeneinander gepresst werden. Somit wird vermieden, dass bei Entkopplung des Kupplungselements und Gegenkupplungselements Fluidrückstände an den Oberflächen des Kupplungselementdichtelements und/oder des Gegenkupplungselementdichtelements auftreten.

Merkmale, Zweckmässigkeiten und Vorteile der Erfindung werden nachfolgend auch anhand von Ausführungsbeispielen unter Bezugnahme auf die Zeichnungen beschrieben.

Es zeigt
Fig. 1 eine schematische Querschnittsansicht eines Kupplungselements zur Verwendung in einem Kupplungssystem in einer Ebene parallel zur Längsachse des Kupplungselements gemäß einer exemplarischen ersten Ausführungsform des Kupplungselements im dekonnektierten Zustand;
Fig. 2 eine schematische Querschnittsansicht eines Gegenkupplungselements zur Verwendung in einem Kupplungssystem in einer Ebene parallel zur Gegenkupplungselementlängsachse des Gegenkupplungselements gemäß einer exemplarischen ersten Ausführungsform des Gegenkupplungselements im dekonnektierten Zustand;
Fig. 3 eine schematische Querschnittsansicht eines Kupplungssystems in einer Ebene parallel zur Längsachse bzw. Gegenkupplungselementlängsachse gemäß der exemplarischen ersten Ausführungsformen des Kupplungselements und des Gegenkupplungselements im konnektierten Zustand;
Fig. 4 eine Übersicht aller Außenansichten des Kupplungssystems gemäß den Figuren 1 bis 3 sowie die Schnittansicht entlang der Schnittlinie A-A, eine perspektivische Ansicht und eine Explosionsdarstellung im dekonnektierten Zustand; sowie
Fig. 5 eine Übersicht aller Außenansichten des Kupplungssystems gemäß den Figuren 1 bis 3 sowie die Schnittansicht entlang der Schnittlinien A-A und eine perspektivische Ansicht im konnektierten Zustand.

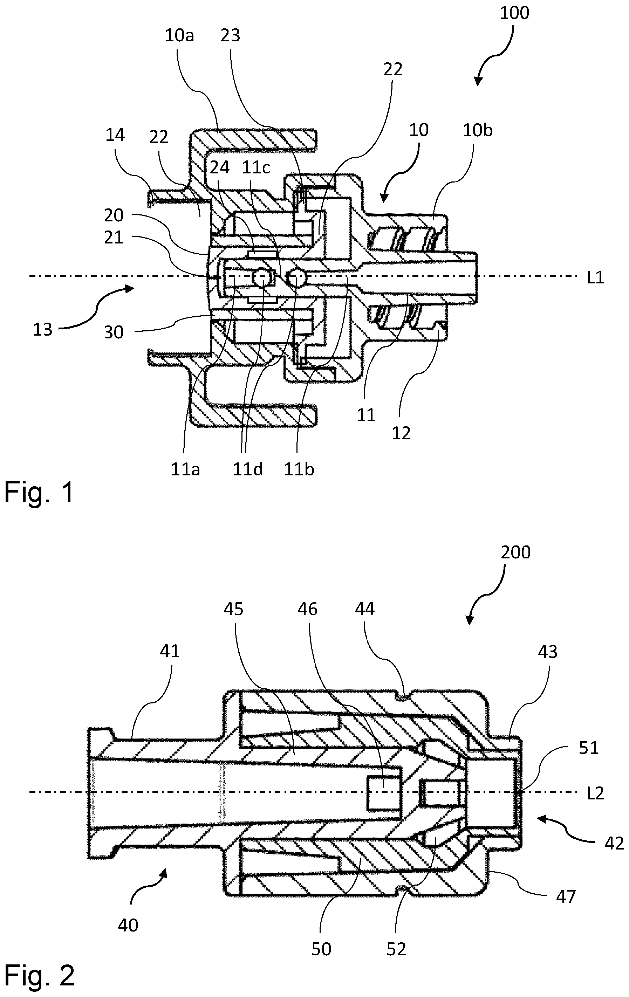

Figur 1 zeigt eine schematische Querschnittsansicht eines Kupplungselements 100 zur Verwendung in einem Kupplungssystem 300 in einer Ebene parallel zur Längsachse L1 des Kupplungselements 100, die sich von einem Fluidanschluss 12 in Richtung einer Kupplungsseite 13 des Kupplungselementgehäuses 10 erstreckt. Neben dem Kupplungselementgehäuse 10 umfasst das Kupplungselement 100 ein Kupplungselementdichtelement 20 mit einer reversibel öffenbaren und schließbaren Kupplungselementdichtelementöffnung 21. Das Kupplungselementdichtelement 20 umschließt ein der Kupplungsseite 13 zugewandtes Ende des Fluidkanals 11, der durch das Kupplungselementgehäuse 10 gebildet wird. Die Kupplungselementdichtelementöffnung 21 ist dabei in einem Bereich der der Kupplungsseite 13 zugewandten Öffnung des Fluidkanals 11 angeordnet. Zudem ist der Fluidkanal 11 über eine Trennwand 11c in einen kupplungsseitenseitigen Fluidkanalabschnitt 11a und einen fluidanschlussseitigen Fluidkanalabschnitt 11b unterteilt. Der kupplungsseitige Fluidkanalabschnitt 11a und der fluidanschlussseitige Fluidkanalabschnitt 11b weisen jeweils eine seitliche Fluidkanalabschnittsöffnung 11d auf, über die sie in fluider Verbindung stehen können.

In der dargestellten Ausführungsform erstreckt sich das Kupplungselementdichtelement 20 ausgehend der Kupplungsseite 13 in Richtung der Längsachse L1 über beide Fluidkanalabschnittsöffnungen 11d. In einer Position des im Folgenden noch beschriebenen Aktivierungselements 30, in der das Kupplungselementdichtelement 20 bzw. Teile hiervon nicht bewegt wurden, das heißt, keine Zugkraft aufgebracht wurde, dichtet ein fluidanschlussseitigen Kupplungselementdichtelementabschnitt des Kupplungselementdichtelements 20 die Fluidkanalabschnittsöffnung 11d des fluidanschlussseitigen Fluidkanalabschnitts 11b ab. Zudem umfasst das Kupplungselementdichtelement 20 eine Fluidkammer 24, die sich zwischen dem fluidanschlussseitigen Kupplungselementdichtelementabschnitt und einem kupplungsseitenseitigen Kupplungselementdichtelementabschnitt des Kupplungselementdichtelements 20 in Bezug auf die Längsachse L1 radial um den Fluidkanal 11 erstreckt, also diesen in tangentialer Richtung umläuft. Die kupplungsseitenseitige Fluidkanalabschnittsöffnung 11d des kupplungsseitenseitigen Fluidkanalabschnitts11a ist im Bereich der Fluidkammer 24 angeordnet.

Um das Kupplungselementdichtelement 20 im Kupplungselementgehäuse 10 zu halten, weist das Kupplungselementdichtelement 20 einen sich in Bezug auf die Längsachse L1 radiale nach außen erstreckenden Befestigungsabschnitt 23 auf, dessen äußeres Ende im Kupplungselementgehäuse 10 befestigt wird. Hierzu wird das Kupplungselementgehäuse 10 beispielsweise, wie in dieser Ausführungsform, aus einem kupplungsseitenseitigen Gehäuseabschnitt 10a und einem fluidanschlussseitigen Gehäuseabschnitt 10b ausgebildet, die den Befestigungsabschnitt 23, beispielsweise durch gegenseitiges Verschrauben, zwischen zwei sich gegenüberliegenden Abschnitten einklemmen.

Zur reversiblen Öffnung der Kupplungselementdichtelementöffnung 21 um fasst das Kupplungselement 100 ein Aktivierungselement 30. Das Aktivierungselement 30 ist ringförmig ausgebildet und wird in Bezug auf die Längsachse L1 in radialer Richtung gesehen zwischen dem den Fluidkanal 11 umgebenden Kupplungselementdichtelement 20 und einer äußeren Kupplungselementgehäusewand angeordnet. In einer Position des Aktivierungselements 30 mit maximalem Abstand zum Fluidanschluss, hier einem dekonnektierten Zustand, bildet das der Kupplungsseite 13 zugewandte Ende des Aktivierungselements 30 zusammen mit der der Kupplungsseite 13 zugewandten Oberfläche des Kupplungselementdichtelements 20 eine kupplungsseitenseitige Stirnfläche aus. Die Wanddicke des Aktivierungselements entspricht im Wesentlichen des Abstands zwischen der radialen Außenfläche des Kupplungselementdichtelements 20 und der äußeren Kupplungselementgehäusewand zur Aufnahme des Aktivierungselements 30. Der Begriff "im Wesentlichen" ist insbesondere darauf bezogen, dass der Innendurchmesser des Aktivierungselements 30 geringfügig kleiner ausgebildet sein kann als der radiale Außendurchmesser des Kupplungselementdichtelements 20 vor einem Aufsetzen des Aktivierungselements 30. Hierdurch entsteht mit Aufsetzen des Aktivierungselements eine Flächenpressung, die das Aktivierungselement 30 auf dem Kupplungselementdichtelement 20 hält. Das Aktivierungselement 30 erstreckt sich in der dargestellten Ausführungsform im dekonnektierten Zustand von der kupplungsseitenseitigen Stirnfläche in Richtung des Fluidanschlusses 12 bis zu einem maximalen Abstand hierzu, der eine ausreichende Bewegung in Richtung des Fluidanschlusses 12 ermöglicht, wobei Das Aktivierungselement 30 auch die Fluidabschnittsöffnung 11d des fluidanschlussseitigen Fluidkanalabschnitts 11b überdeckt. Durch die angeführte Überdeckung wird die Abdichtung über den fluidanschlussseitigen Kupplungselementdichtelementabschnitt unterstützt, insbesondere bei Vorsehung der genannten Flächenpressung. Des Weiteren greift das dem Fluidanschluss 12 zugewandte Ende des Aktivierungselements 30 in einen in Bezug auf die Längsachse L1 radial nach außen weisenden Vorsprung 22 des Kupplungselementdichtelements 20 ein.

Als Nächstes wird ein erfindungsgemäßes Gegenkupplungselement zur Verwendung in einem Kupplungssystem in Bezug auf Figur 2 anhand einer schematische Querschnittsansicht eines Gegenkupplungselements 200 in einer Ebene parallel zur Gegenkupplungselementlängsachse L2 des Gegenkupplungselements 200, die sich von einem Gegenkupplungselementfluidanschluss 41 des Gegenelementkupplungsgehäuses 40 in Richtung einer Gegenkupplungsseite 42 erstreckt. Neben dem Gegenkupplungselementgehäuse 40 umfasst das Gegenkupplungselement 200 ein Gegenkupplungselementdichtelement 50, das in dem Gegenkupplungselementgehäuse 40 angeordnet ist und zusammen mit dem Gegenkupplungselementgehäuse 40 zumindest einen Teil einer gegenkupplungsseitigen Stirnfläche des Gegenkupplungselements 200 ausbildet.

Das Gegenkupplungselementgehäuse 40 weist ein gegenkupplungsseitenseitiges Ende 43 auf, das sich in Bezug auf die Gegenkupplungselementlängsachse L2 in radialer Richtung an das Gegenkupplungselementdichtelement 50 anschließt und dieses umgibt. Dieses gegenkupplungsseitenseitige Ende 43 bildet in Bezug auf die Gegenkupplungselementlängsachse L2 einen axialen Vorsprung aus und bildet zudem mit einem sich daran anschließenden Gehäuseabschnitt des Gegenkupplungselementgehäuses 40 einen Schulterabschnitt 47, der als Anschlag für einen korrespondierenden Kupplungselementabschnitt des Kupplungselementgehäuses 10 fungiert. Darüber hinaus weist das Gegenkupplungselementgehäuse 40 in dem sich an den durch das gegenkupplungsseitenseitige Ende 43 gebildeten axialen Vorsprung anschließenden Gehäuseabschnitt des Gegenkupplungselementgehäuses 40 eine Gegenkupplungselementhaltestruktur 44 auf, die in der vorliegenden Ausführungsform über eine umlaufende Nut gebildet wird, in die die Schnapphaken der Haltestruktur 14 des in Figur 1 gezeigten Kupplungselements eingreifen können. Vom Gegenkupplungselementfluidanschluss 41 aus erstreckt sich ein Gegenkupplungselementfluidkanal 45 in das Gegenkupplungselementgehäuses 40 hinein. Das gegenkupplungsseitenseitige Fluidkanalende ist in Bezug auf die Gegenkupplungselementlängsachse L2 axial von der Gegenkupplungsseite 42 beabstandet und geschlossen, wobei eine seitliche Gegenkupplungselementfluidkanalöffnung 46 vorgesehen ist, die im dekonnektierten Zustand durch das Gegenkupplungselementdichtelement 50 abgedichtet wird.

Das in Figur 2 dargestellte Gegenkupplungselementdichtelement 50 weist in der der Gegenkupplungsseite 42 zugewandten Oberfläche eine reversibel öffenbare und schließbare Gegenkupplungselementdichtelementöffnung 51 auf, die in einem dekonnektierten Zustand geschlossen ist. Das Gegenkupplungselementdichtelement 50 ist insgesamt elastisch ausgebildet und stützt sich an seinem fluidanschlussseitigen Ende gegen einen ihm zugewandten Gehäuseabschnitt des Gegenkupplungselementgehäuses 40 ab, wobei es den Gegenkupplungselementfluidkanal 45 umschließt. Im dekonnektierten Zustand bildet das Gegenkupplungselementdichtelement 50 zwischen der der Gegenkupplungsseite 42 zugewandten Oberfläche des Gegenkupplungselementdichtelements 50 und dem der Gegenkupplungsseite 42 zugewandten geschlossenen Ende des Gegenkupplungselementfluidkanals 45 eine Gegenkupplungselementdichtelementfluidkammer 52 aus.

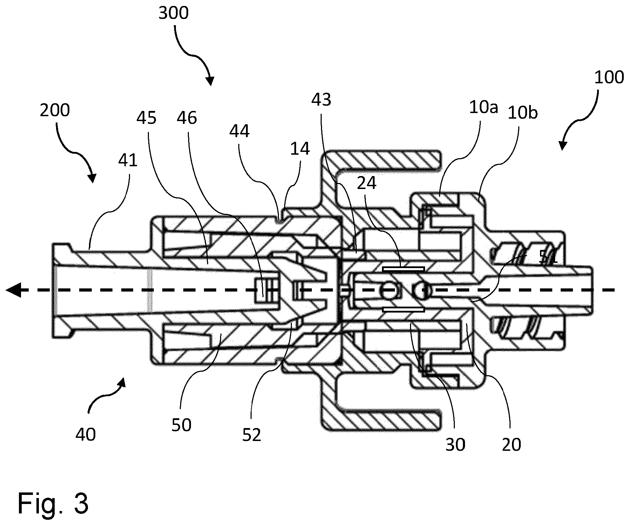

Figur 3 zeigt eine schematische Querschnittsansicht eines Kupplungssystems 300 in einer Ebene parallel zur Längsachse L1 bzw. Gegenkupplungselementlängsachse L2, das die exemplarischen Ausführungsformen des Kupplungselements 100 gemäß Figur 1 und des Gegenkupplungselements 200 gemäß Figur 2 umfasst und im konnektierten Zustand darstellt.

Der konnektierte Zustand wird erreicht, indem das Gegenkupplungselement 200 in Richtung der Längsachse L1 bzw. Gegenkupplungselementlängsachse L2 in Richtung des Fluidanschlusses 12 und/oder das Kupplungselement 100 in Richtung der Längsachse L1 bzw. Gegenkupplungselementlängsachse L2 in Richtung des Gegenkupplungselementfluidanschlusses 41 bewegt wird. Der durch das gegenkupplungsseitenseitige Ende 43 gebildete axiale Vorsprung wird hierüber in den Abstand zwischen dem Kupplungselementdichtelement 20 und dem Kupplungselementgehäuse 40 bewegt und schiebt dadurch das Aktivierungselement 30 von einer Position mit maximalem Abstand zum Fluidanschluss 12 in Richtung des Fluidanschlusses 12. Hierdurch wird das Kupplungselementdichtelement 20 aus elastischem Material gedehnt, wodurch eine Zugkraft auf den die Kupplungselementdichtelementöffnung 21 umgebenden Bereich ausgeübt wird, die die Kupplungselementdichtelementöffnung 21 öffnet. Durch die Dehnung des Kupplungselementdichtelements 20 dehnt sich gleichzeitig die Fluidkammer 41 in axialer Richtung in Bezug auf die Längsachse L1 in Richtung des Fluidanschlusses 12 aus, so dass die Fluidkanalabschnittsöffnung 11d des fluidanschlussseitigen Fluidkanalabschnitts 11b nicht mehr abgedichtet wird. Im konnektierten Zustand, in dem der Schulterabschnitt 47 auf dem Kupplungselementgehäuse 10 aufliegt und die Haltestruktur 14 des Kupplungselements 100 in die Gegenkupplungselementhaltestruktur 44 eingreift, kann somit ein Fluid über den fluidanschlussseitigen Fluidkanalabschnitt 11b, die Fluidkanalabschnittsöffnung 11d des fluidanschlussseitigen Fluidkanalabschnitts 11b, die Fluidkammer 24, die Fluidkanalabschnittsöffnung 11d des kupplungsseitenseitigen Fluidkanalabschnitts 11a, den kupplungsseitenseitigen Fluidkanalabschnitt 11a und die Kupplungselementdichtelementöffnung 21 mit dem Gegenkupplungselement 200 ausgetauscht werden, wie dies durch die Pfeillinie in Figur 3 angezeigt wird.

Zum Fluidaustausch des Gegenkupplungselements 200 mit dem Kupplungselement 100 wird die der Gegenkupplungssseite 42 zugewandte Oberfläche mit der Gegenkupplungselementdichtelementöffnung 51 über die der Kupplungsseite 13 zugewandte Oberfläche des Kupplungselementdichtelements 20 bzw. mittelbar durch das der Kupplungsseite 13 zugewandte Ende des Fluidkanals 11 in Richtung des Gegenkupplungselementfluidkanals 41 gedrückt. Hierüber wird die Gegenkupplungselementdichtelementöffnung 51 geöffnet. Gleichzeitig wird das Gegenkupplungselementdichtelement 50 in Richtung des Gegenkupplungselementfluidanschlusses 41 komprimiert, wodurch die Gegenkupplungselementdichtelementfluidkammer 52 in Richtung des Gegenkupplungselementfluidanschlusses 41 bewegt wird, um im konnektierten Zustand zumindest einen Bereich der der Gegenkupplungselementfluidkanalöffnung 46 zu überdecken, so dass ein Fluidaustausch ermöglicht wird. Somit kann gemäß der Pfeillinie der Fluidaustausch im konnektierten Zustand weiter über die Gegenkupplungselementdichtelementöffnung 51 die Gegenkupplungselementfluidkammer 52, die Gegenkupplungselementfluidkanalöffnung 46 und den Gegenkupplungselementfluidkanal 41 erfolgen.

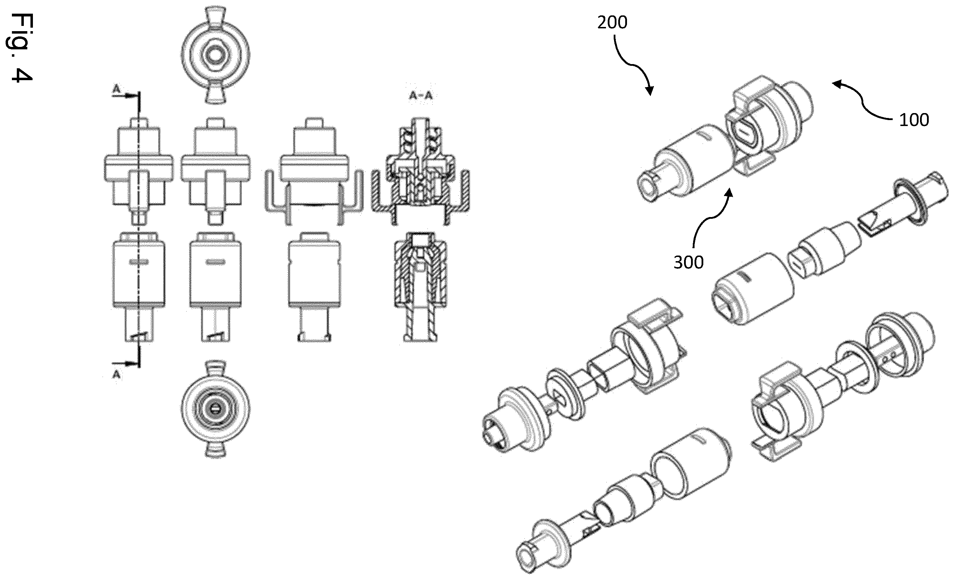

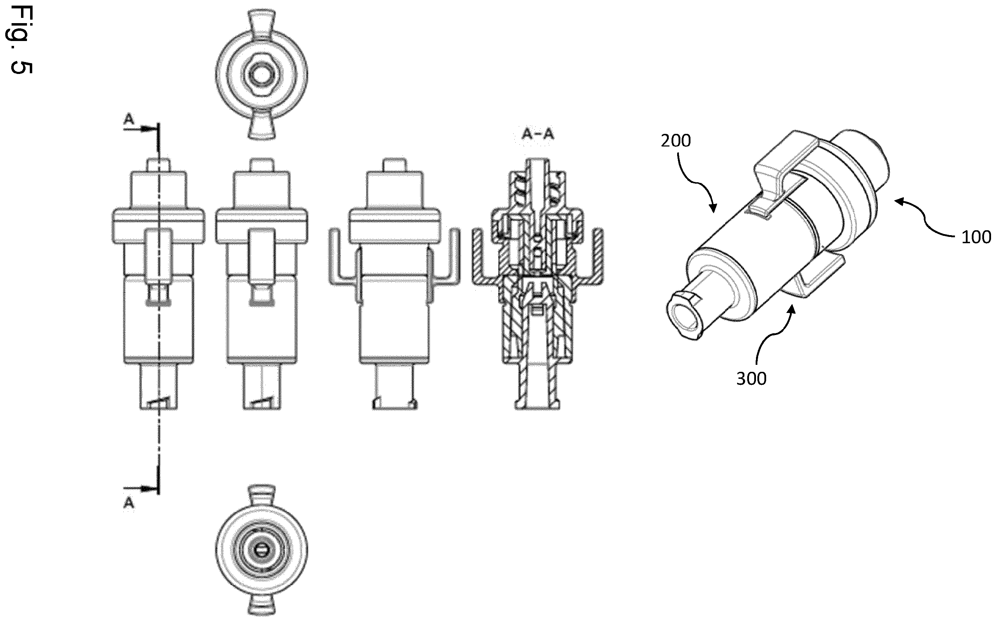

Die Figuren 4 und 5 zeigen die vorstehend beschriebene Ausführungsform zum Kupplungssystem 300 nochmals in einer Übersicht aller Ansichten im dekonnektierten Zustand gemäß Figur 4 und im konnektierten Zustand gemäß Figur 5. Hieraus ergeben sich weitere Ausgestaltungsmerkmale der beschriebenen Ausführungsform.

Die Erfindung ist nicht auf die beschriebenen Ausführungsformen beschränkt. Beispielsweise sind die in den Figuren gezeigte, der Kupplungsseite zugewandte Oberfläche des Kupplungselementdichtelements konvex und die der Gegenkupplungsseite zugewandte Oberfläche der Gegenkupplungselements hierzu korrespondierend konkav ausgebildet. Auch wenn dies die jeweilige Öffnungsausbildung unterstützt, können alternativ gerade Oberflächen oder anderweitige Konturierungen vorgesehen werden. Wie bereits angeführt, müssen das Kupplungselementdichtelement und das Gegenkupplungselementdichtelement auch nicht, wie in den beschriebenen Ausführungsformen, vollständig aus elastischem Material gebildet sein, sondern können dieses nur abschnittsweise aufweisen. Insbesondere können alternativ oder ergänzend zur Verwendung eines elastischen Materials auch elastische Strukturen eingesetzt werden, wie beispielsweise Federgelenke oder dergleichen.

### Liste der Bezugszeichen

- 10: Kupplungselementgehäuse
- 10a: kupplungsseitenseitiger Gehäuseabschnitt
- 10b: fluidanschlussseitiger Gehäuseabschnitt
- 11: Fluidkanal
- 11a: kupplungsseitenseitiger Fluidkanalabschnitt
- 11b: fluidanschlussseitiger Fluidkanalabschnitt
- 11c: Trennwand
- 11d: Fluidkanalabschnittsöffnung
- 12: Fluidanschluss
- 13: Kupplungsseite
- 14: Haltestruktur
- 20: Kupplungselementdichtelement
- 21: Kupplungselementdichtelementöffnung
- 22: Vorsprung
- 23: Befestigungsabschnitt
- 24: Fluidkammer
- 30: Aktivierungselement
- 40: Gegenkupplungselementgehäuse
- 41: Gegenkupplungselementfluidanschluss
- 42: Gegenkupplungsseite
- 43: gegenkupplungsseitenseitiges Ende
- 44: Gegenkupplungselementhaltestruktur
- 45: Gegenkupplungselementfluidkanal
- 46: Gegenkupplungselementfluidkanalöffnung
- 47: Schulterabschnitt
- 50: Gegenkupplungselementdichtelement
- 51: Gegenkupplungselementdichtelementöffnung
- 52: Gegenkupplungselementdichtelementfluidkammer
- 100: Kupplungselement
- 200: Gegenkupplungselement
- 300: Kupplungssystem
- L1: Längsachse (Kupplungselement)
- L2: Gegenkupplungselementlängsachse

## Patentansprüche

1. Kupplungssystem (300) für ein geschlossenes Fluidtransfersystem, umfassend zumindest ein Kupplungselement (100) sowie zumindest ein Gegenkupplungselement (200),
wobei das Kupplungselement (100)
ein Kupplungselementgehäuse (10) mit einem Fluidanschluss (12) und einer Kupplungsseite (13), wobei das Kupplungselementgehäuse (10) eine sich von dem Fluidanschluss (12) in Richtung der Kupplungsseite (13) erstreckende Längsachse (L1) aufweist,
einen Fluidkanal (11), der sich in Richtung der Längsachse (L1) vom Fluidanschluss (12) in das Kupplungselementgehäuse (10) erstreckt,
ein Kupplungselementdichtelement (20), das eine der Kupplungsseite (13) zugewandte Fluidkanalöffnung des Fluidkanals (11) überdeckt, wobei das Kupplungselementdichtelement (20) zumindest eine reversibel öffenbare und schließbare Kupplungselementdichtelementöffnung (21) im Bereich der Überdeckung der der Kupplungsseite (13) zugewandten Fluidkanalöffnung aufweist und zumindest ein sich an die Kupplungselementdichtelementöffnung (21) anschließender und diese umfassender Bereich, insbesondere das gesamte Kupplungselementdichtelement (20), aus einem elastischen Material ausgebildet ist, und
zumindest ein Aktivierungselement (30), das an dem Kupplungselementdichtelement (20) angreift und in Richtung der Längsachse (L1) im Kupplungselementgehäuse (10) zwischen einer Position mit maximalem Abstand zum Fluidanschluss (12) und einer Position mit minimalem Abstand zum Fluidanschluss (12) bewegbar ist, wobei das Aktivierungselement derart konfiguriert ist, dass die Kupplungselementdichtelementöffnung (21) des Kupplungselementdichtelements (20) in der Position des Aktivierungselements (30) mit maximalem Abstand zum Fluidanschluss (12) geschlossen ist, und dass die Kupplungselementdichtelementöffnung (21) in der Position des Aktivierungselements mit minimalem Abstand zu Fluidanschlusses (12) geöffnet ist,
umfasst, und
wobei das Gegenkupplungselement (200) zur Kupplung mit dem Kupplungselement (100)
ein Gegenkupplungselementgehäuse (40) mit einem Gegenkupplungselementfluidanschluss (41) und einer Gegenkupplungsseite (42), wobei das Gegenkupplungselementgehäuse (40) eine sich von dem Gegenkupplungselementfluidanschluss (41) in Richtung der Gegenkupplungsseite (42) erstreckende Gegenkupplungselementlängsachse (L2) aufweist, und
ein Gegenkupplungselementdichtelement (50), das in dem Gegenkupplungselementgehäuse (40) angeordnet ist und zusammen mit dem Gegenkupplungselementgehäuse (40) zumindest einen Teil einer gegenkupplungsseitigen Stirnfläche des Gegenkupplungselements (200) ausbildet,
umfasst,
wobei das Gegenkupplungselementdichtelement (50) im Bereich der gegenkupplungsseitigen Stirnfläche eine reversibel öffenbare und schließbare Gegenkupplungselementdichtelementöffnung (51) aufweist und zumindest ein sich an die Gegenkupplungselementdichtelementöffnung (51) anschließender und diese umfassender Bereich, insbesondere das gesamte Gegenkupplungselementdichtelement (50), aus einem elastischen Material ausgebildet ist, und
wobei das Gegenkupplungselement (200) derart konfiguriert ist, dass es das Aktivierungselement (30) bei einer Kupplung in die Position mit minimalem Abstand zum Fluidanschluss (12) bewegt, wobei eine durch die Bewegung des Aktivierungselements (30) bedingte Öffnung der Kupplungselementdichtelementöffnung (21) erst erfolgt, wenn die Kupplungselementdichtelementöffnung (21) über das Gegenkupplungselement (200) nach außen abgedichtet ist, und
dadurch charakterisiert, dass die reversibel öffenbare und schließbare Gegenkupplungselementdichtelementöffnung (51) in einem konnektierten Zustand durch das Kupplungselementdichtelement (20) geöffnet wird.

2. Kupplungssystem (300) nach Anspruch 1, wobei das Kupplungselementdichtelement (20) ausgehend von dem Bereich der Überdeckung der der Kupplungsseite (13) zugewandten Fluidkanalöffnung den Fluidkanal (11) in Richtung des Fluidanschlusses (12) zumindest über einen vorbestimmten Abschnitt in Bezug auf die Längsachse (L1) radial umgibt und einen in Bezug auf die Längsachse (L1) radial nach außen weisenden Vorsprung (22), insbesondere an einem dem Fluidanschluss (12) zugewandten Ende des Kupplungselementdichtelements (20), aufweist, an dem ein dem Fluidanschluss (12) zugewandtes Ende oder ein in Bezug auf die Längsachse (L1) radial nach innen weisender Vorsprung des Aktivierungselements (30) angreift.

3. Kupplungssystem (300) nach Anspruch 1 oder 2, wobei der Fluidkanal (11) zwei durch eine Trennwand (11c) voneinander getrennte Fluidkanalabschnitte (11a, 11b) umfasst, die jeweils eine seitliche Fluidkanalabschnittsöffnung (11d) aufweisen, und wobei das Kupplungselementdichtelement (20) eine Fluidkammer (24) aufweist, die sich zwischen einem kupplungsseitigen Kupplungselementdichtelementabschnitt und einem fluidanschlussseitigen Kupplungselementdichtelementabschnitt in Bezug auf die Längsachse (L1) radial um den Fluidkanal (11) erstreckt, wobei der fluidanschlussseitige Kupplungselementdichtelementabschnitt in einem Zustand, in dem sich das Aktivierungselement (30) in der Position mit maximalem Abstand zum Fluidanschluss (12) befindet, zumindest die Fluidkanalabschnittsöffnung (11d) des fluidanschlussseitigen Fluidkanalabschnitts (11b) abdichtet und sich die Fluidkammer (24) sich in einem Zustand, in dem sich das Aktivierungselement (30) in der Position mit minimalen Abstand zum Fluidanschluss (12) befindet, über die seitliche Fluidkanalabschnittsöffnung (11d) des kupplungsseitenseitigen Fluidkanalabschnitts (11a) und die seitliche Fluidkanalabschnittsöffnung (11d) des fluidanschlussseitigen Fluidkanalabschnitts (11b) erstreckt, um über die Fluidkammer (24) eine fluide Verbindung zwischen dem kupplungsseitenseitigen Fluidkanalabschnitt (11a) und dem fluidanschlussseitigen Fluidkanalabschnitt (11b) auszubilden.

4. Kupplungssystem (300) nach Anspruch 2 oder 3, wobei der in Bezug auf die Längsachse (L1) nach außen weisende Vorsprung (22) des Kupplungselementdichtelements (20) einen Befestigungsabschnitt (23) aufweist, der im Kupplungselementgehäuse (10) befestigt ist.

5. Kupplungssystem (300) nach Anspruch 4, wobei das Kupplungselementgehäuse (10) entlang der Längsachse (L1) einen kupplungsseitenseitigen Gehäuseabschnitt (10a) und einen fluidanschlussseitigen Gehäuseabschnitt (10b) umfasst, zwischen denen der Befestigungsabschnitt (23) des Kupplungselementdichtelements (20) gehalten wird.

6. Kupplungssystem (300) nach einem der Ansprüche 1 bis 5, wobei das Aktivierungselement (30) ringförmig ausgebildet ist, wobei insbesondere der Ring des Aktivierungselements (30) am zum Fluidanschluss (12) weisenden Ende des Kupplungselementdichtelements (20) angreift.

7. Kupplungssystem (300) nach einem der Ansprüche 1 bis 6, wobei das Kupplungselementgehäuse (10) auf der Kupplungsseite (13) zumindest eine Haltestruktur (14), insbesondere eine in Bezug auf die Längsachse (L1) auf der Kupplungsseite (13) axial über das Kupplungselementdichtelement (20) vorstehende Haltestruktur (14), aufweist, über die ein Gegenkupplungselement (200) in einem konnektierten Zustand gehalten werden kann.

8. Kupplungssystem (300) nach Anspruch 7, wobei die Haltestruktur (14) über zumindest zwei Haltearme mit Schnapphaken oder ringförmig ausgebildet wird.

9. Kupplungssystem (300) nach einem der vorherigen Ansprüche, wobei ein gegenkupplungsseitenseitiges Ende (43) des Gegenkupplungselementgehäuses (40) an dem zumindest einen Aktivierungselement (30) des Kupplungselements (100) angreifen kann und mit diesem zusammen in Richtung des Fluidanschlusses (12) des Kupplungselements (100) in das Kupplungselementgehäuse (10) bewegbar ist.

10. Kupplungssystem (300) nach Anspruch 9, wobei das gegenkupplungsseitenseitige Ende (43) des Gegenkupplungselementgehäuses (40) als in Bezug auf die Gegenkupplungselementlängsachse (L2) axialer Vorsprung ausgebildet ist und mit einem sich daran anschließenden Gehäuseabschnitt des Gegenkupplungselementgehäuses (40) einen Schulterabschnitt (47) bildet.

11. Kupplungssystem (300) der vorherigen Ansprüche, wobei das Gegenkupplungselementgehäuse (40) an einer sich in Bezug auf die Gegenkupplungselementlängsachse (L2) axial erstreckenden Außenfläche eine Gegenkupplungselementhaltestruktur (44) aufweist, über den das Gegenkupplungselement (200) in einem konnektierten Zustand mit dem Kupplungselement (100) gehalten werden kann.

12. Kupplungssystem nach einem der vorherigen Ansprüche, wobei das Gegenkupplungselementgehäuse (40) einen Gegenkupplungselementfluidkanal (45) mit zumindest einer seitlichen Gegenkupplungselementfluidkanalöffnung (46) umfasst, der sich in Richtung der Gegenkupplungselementlängsachse (L2) vom Gegenkupplungselementfluidanschluss (41) in Richtung der Gegenkupplungsseite (42) im Gegenkupplungselementgehäuse (40) erstreckt, und wobei das Gegenkupplungselementdichtelement (51) in einem dekonnektierten Zustand die seitliche Gegenkupplungselementfluidkanalöffnung (46) abdichtet, wobei das Gegenkupplungselementdichtelement (51) zwischen der Abdichtung und der Gegenkupplungselementdichtelementöffnung (51) eine Gegenkupplungselementdichtelementfluidkammer (52) aufweist, die in Richtung der Gegenkupplungselementlängsachse (L2) relativ zum Gegenkupplungselementfluidkanal (45) bewegbar ist, um im konnektierten Zustand über die seitliche Gegenkupplungselementfluidkanalöffnung (46) eine fluide Verbindung zum Inneren des Gegenkupplungselementfluidkanals (45) auszubilden.

## Claims

1. Coupling system (300) for a closed fluid transfer system, comprising at least one coupling element (100) and at least one counter-coupling element (200),
wherein the coupling element (100) comprises
a coupling element housing (10) with a fluid port (12) and a coupling side (13), wherein the coupling element housing (10) has a longitudinal axis (L1) extending from the fluid port (12) in the direction of the coupling side (13),
a fluid channel (11), which extends in the direction of the longitudinal axis (L1) from the fluid port (12) into the coupling element housing (10),
a coupling element sealing element (20), which covers a fluid channel opening of the fluid channel (11) facing the coupling side (13), wherein the coupling element sealing element (20) has at least one reversibly openable and closable coupling element sealing element opening (21) in the region of the covering of the fluid channel opening facing the coupling side (13) and at least one region adjoining and encompassing the coupling element sealing element opening (21), in particular the entire coupling element sealing element (20), is formed from an elastic material, and
at least one activating element (30), which engages with the coupling element sealing element (20) and can be moved in the direction of the longitudinal axis (L1) in the coupling element housing (10) between a position with a maximum distance from the fluid port (12) and a position with a minimum distance from the fluid port (12), wherein the activating element is designed such that the coupling element sealing element opening (21) of the coupling element sealing element (20) is closed in the position of the activating element (30) with a maximum distance from the fluid port (12), and the coupling element sealing element opening (21) is open in the position of the activating element with a minimum distance from the fluid port (12),
and
wherein the counter-coupling element (200) comprises for coupling to the coupling element (100)
a counter-coupling element housing (40) with a counter-coupling element fluid port (41) and a counter-coupling side (42), wherein the counter-coupling element housing (40) has a counter-coupling element longitudinal axis (L2) extending from the counter-coupling element fluid port (41) in the direction of the counter-coupling side (42), and
a counter-coupling element sealing element (50), which is arranged in the counter-coupling element housing (40) and together with the counter-coupling element housing (40) forms at least part of a counter-coupling side end face of the counter-coupling element (200),
wherein the counter-coupling element sealing element (50) has a reversibly openable and closable counter-coupling element sealing element opening (51) in the region of the counter-coupling side end face and at least one region adjoining and encompassing the counter-coupling element sealing element opening (51), in particular the entire counter-coupling element sealing element (20), is formed from an elastic material, and
wherein the counter-coupling element (200) is designed in such a way that it moves the activating element (30) into the position with a minimum distance from the fluid port (12) during coupling, wherein opening of the coupling element sealing element opening (21) caused by the movement of the activating element (30) only occurs if the coupling element sealing element opening (21) is sealed to the outside via the counter-coupling element (200), and **characterised in that**
the reversibly openable and closable counter-coupling element sealing element opening (51) is opened in a connected state by the coupling element sealing element (20).

2. Coupling system (300) according to claim 1, wherein the coupling element sealing element (20), starting from the region of the covering of the fluid channel opening facing the coupling side (13), radially surrounds the fluid channel (11) in the direction of the fluid port (12) at least over a predetermined section in relation to the longitudinal axis (L1) and has a projection (22) pointing radially outwards with respect to the longitudinal axis (L1), in particular on an end of the coupling element sealing element (20) facing the fluid port (12), with which an end facing the fluid port (12) or a projection of the activating element (30) pointing radially inwards in relation to the longitudinal axis (L1) engages.

3. Coupling system (300) according to claim 1 or 2, wherein the fluid channel (11) comprises two fluid channel sections (11a, 11b) separated from one another by a partition (11c) and which respectively have a lateral fluid channel section opening (11d), and wherein the coupling element sealing element (20) has a fluid chamber (24), which extends radially in relation to the longitudinal axis (L1) around the fluid channel (11) between a coupling element sealing element section on the coupling side and a coupling element sealing element section on the fluid port side, wherein the coupling element sealing element section on the fluid port side seals at least the fluid channel section opening (11d) of the fluid channel section (11b) on the fluid port side in a state in which the activating element (30) is in the portion with a maximum distance from the fluid port (12), and the fluid chamber (24) extends over the lateral fluid channel section opening (11d) of the fluid channel section (11a) on the coupling side side and the lateral fluid channel section opening (11d) of the fluid channel section (11b) on the fluid port side in a state in which the activating element (30) is in the position with a minimum distance to the fluid port (12) in order to form a fluid connection between the fluid channel section (11a) on the coupling side side and the fluid channel section (11b) on the fluid port side via the fluid chamber (24).

4. Coupling system (300) according to claim 2 or 3, wherein the projection (22) of the coupling element sealing element (20) pointing outwards in relation to the longitudinal axis (L1) has a fastening section (23), which is fastened in the coupling element housing (10).

5. Coupling system (300) according to claim 4, wherein the coupling element housing (10) comprises a housing section (10a) on the coupling side side and a housing section (10b) on the fluid port side along the longitudinal axis (L1), between which the fastening section (23) of the coupling element sealing element (20) is held.

6. Coupling system (300) according to one of claims 1 to 5, wherein the activating element (30) is formed in the shape of a ring, wherein in particular the ring of the activating element (30) engages with the end of coupling element sealing element (20) pointing towards the fluid port (12).

7. Coupling system (300) according to one of claims 1 to 6, wherein the coupling element housing (10) has at least one retaining structure (14) on the coupling side (13), in particular a retaining structure (14) projecting axially beyond the coupling element sealing element (20) in relation to the longitudinal axis (L1) on the coupling side (13), by means of which a counter-coupling element (200) can be retained in a connected state.

8. Coupling system (300) according to claim 7, wherein the retaining structure (14) is formed by at least two retaining arms with snap hooks or in the shape of a ring.

9. Coupling system (300) according to one of the preceding claims, wherein an end (43) of the counter-coupling element housing (40) on the counter-coupling side side can engage with the at least one activating element (30) of the coupling element (100) and can be moved together with it in the direction of the fluid port (12) of the coupling element (100) into the coupling element housing (10).

10. Coupling system (300) according to claim 9, wherein the end (43) of the counter-coupling element housing (40) on the counter-coupling side side is designed as an axial projection in relation to the counter-coupling element longitudinal axis (L2) and forms a shoulder section (47) with an adjoining housing section of the counter-coupling element housing (40).

11. Coupling system (300) according to the preceding claims, wherein the counter-coupling element housing (40) has a counter-coupling element retaining structure (44) on an outer surface extending axially in relation to the counter-coupling element longitudinal axis (L2), via which the counter-coupling element (200) can be held in a connected state with the coupling element (100).

12. Coupling system according to one of the preceding claims, wherein the counter-coupling element housing (40) comprises a counter-coupling element fluid channel (45) with at least one lateral counter-coupling element fluid channel opening (46), which extends in the direction of the counter-coupling element longitudinal axis (L2) from the counter-coupling element fluid port (41) in the direction of the counter-coupling side (42) in the counter-coupling element housing (40), and wherein the counter-coupling element sealing element (51) seals the lateral counter-coupling element fluid channel opening (46) in a disconnected state, wherein the counter-coupling element sealing element (51) has a counter-coupling element sealing element fluid chamber (52) between the seal and the counter-coupling element sealing element opening (51), which can be moved in the direction of the counter-coupling element longitudinal axis (L2) relative to the counter-coupling element fluid channel (45) in order to form a fluid connection to the inside of the counter-coupling element fluid channel (45) in the connected state via the lateral counter-coupling element fluid channel opening (46).

## Revendications

1. Système d'accouplement (300) pour un système fermé de transfert de fluide, comprenant au moins un élément d'accouplement (100) ainsi qu'au moins un élément d'accouplement complémentaire (200),
dans lequel l'élément d'accouplement (100) comporte
un boîtier d'élément d'accouplement (10) ayant un raccord de fluide (12) et un côté d'accouplement (13), dans lequel le boîtier d'élément d'accouplement (10) a un axe longitudinal (L1) s'étendant à partir du raccord de fluide (12) en direction du côté d'accouplement (13),
un canal de fluide (11) qui s'étend dans le boîtier d'élément d'accouplement (10) en direction de l'axe longitudinal (L1) à partir du raccord de fluide (12), un élément d'étanchéité d'élément d'accouplement (20) qui recouvre une ouverture de canal de fluide du canal de fluide (11) faisant face au côté d'accouplement (13), dans lequel l'élément d'étanchéité d'élément d'accouplement (20) comporte au moins une ouverture d'élément d'étanchéité d'élément d'accouplement (13) pouvant être ouverte et fermée de manière réversible dans la zone du recouvrement de l'ouverture de canal de fluide faisant face au côté d'accouplement (13), et au moins une zone, en particulier l'élément d'étanchéité d'élément d'accouplement (20) complet, adjacente à l'ouverture d'élément d'étanchéité d'élément d'accouplement (21) et incluant celle-ci, est formée d'un matériau élastique, et
au moins un élément d'activation (30) qui vient en prise avec l'élément d'étanchéité d'élément d'accouplement (20) et qui est déplaçable en direction de l'axe longitudinal (L1) dans le boîtier d'élément d'accouplement (10) entre une position à distance maximale du raccord de fluide (12) et une position à distance minimale du raccord de fluide (12), dans lequel l'élément d'activation est configuré de telle sorte que l'ouverture d'élément d'étanchéité d'élément d'accouplement (21) de l'élément d'étanchéité d'élément d'accouplement (20) est fermée dans la position de l'élément d'activation (30) à distance maximale du raccord de fluide (12), et que l'ouverture d'élément d'étanchéité d'élément d'accouplement (21) est ouverte dans la position de l'élément d'activation à distance minimale du raccord de fluide (12),
et
dans lequel l'élément d'accouplement complémentaire (200) destiné à l'accouplement avec l'élément d'accouplement (100) comprend
un boîtier d'élément d'accouplement complémentaire (40) ayant un raccord de fluide d'élément d'accouplement complémentaire (41) et un côté d'accouplement complémentaire (42), dans lequel le boîtier d'élément d'accouplement complémentaire (40) a un axe longitudinal d'élément d'accouplement complémentaire (L2) s'étendant à partir du raccord de fluide d'élément d'accouplement complémentaire (41) en direction du côté d'accouplement complémentaire (42), et
un élément d'étanchéité d'élément d'accouplement complémentaire (50) qui est agencé dans le boîtier d'élément d'accouplement complémentaire (40) et forme, en association avec le boîtier d'élément d'accouplement complémentaire (40), au moins une portion d'une face frontale côté accouplement complémentaire de l'élément d'accouplement complémentaire (200),
dans lequel l'élément d'étanchéité d'élément d'accouplement complémentaire (50) comporte une ouverture d'élément d'étanchéité d'élément d'accouplement complémentaire (51) pouvant être ouverte et fermée de manière réversible dans la zone de la face frontale côté accouplement complémentaire, et au moins une zone, en particulier l'élément d'étanchéité d'élément d'accouplement complémentaire (50) complet, adjacente à l'ouverture d'élément d'étanchéité d'élément d'accouplement complémentaire (51) et incluant celle-ci, est formée d'un matériau élastique, et
dans lequel l'élément d'accouplement complémentaire (200) est configuré de telle sorte qu'il amène l'élément d'activation (30) dans la position à distance minimale du raccord de fluide (12) lors d'un accouplement, dans lequel une ouverture de l'ouverture d'élément d'étanchéité d'élément d'accouplement (21) due au mouvement de l'élément d'activation (30) n'a lieu que lorsque l'ouverture d'élément d'étanchéité d'élément d'accouplement complémentaire (21) est rendue étanche par rapport à l'extérieur via l'élément d'accouplement complémentaire (30), et
**caractérisé en ce que**
l'ouverture d'élément d'étanchéité d'élément d'accouplement complémentaire (51) pouvant être ouverte et fermée de manière réversible est ouverte dans un état connecté par l'élément d'étanchéité d'élément d'accouplement (20).

2. Système d'accouplement (300) selon la revendication 1, dans lequel l'élément d'étanchéité d'élément d'accouplement (20), en partant de la zone du recouvrement de l'ouverture de canal de fluide faisant face au côté d'accouplement (13), entoure radialement le canal de fluide (11) en direction du raccord de fluide (12) au moins sur une portion prédéterminée par rapport à l'axe longitudinal (L1) et comporte une saillie (22) dirigée radialement vers l'extérieur par rapport à l'axe longitudinal (L1), en particulier au niveau d'une extrémité de l'élément d'étanchéité d'élément d'accouplement (20) faisant face au raccord de fluide (12), avec laquelle vient en prise une extrémité faisant face au raccord de fluide (12) ou une saillie de l'élément d'activation (30) dirigée radialement vers l'intérieur par rapport à l'axe longitudinal (L1).

3. Système d'accouplement (300) selon la revendication 1 ou 2, dans lequel le canal de fluide (11) comprend deux portions de canal de fluide (11a, 11b) séparées l'une de l'autre par une cloison (11c), qui comportent respectivement une ouverture latérale de portion de canal de fluide (11d), et dans lequel l'élément d'étanchéité d'élément d'accouplement (20) comporte une chambre de fluide (24) qui s'étend radialement par rapport à l'axe longitudinal (L1) autour du canal de fluide (11) entre une portion d'élément d'étanchéité d'élément d'accouplement côté accouplement et une portion d'élément d'étanchéité d'élément d'accouplement côté raccord de fluide, dans lequel la portion d'élément d'étanchéité d'élément d'accouplement côté raccord de fluide rend étanche au moins l'ouverture de portion de canal de fluide (11d) de la portion de canal de fluide côté raccord de fluide (11b) dans un état dans lequel l'élément d'activation (30) est dans la position à distance maximale du raccord de fluide (12), et la chambre de fluide (24) s'étend au-delà de l'ouverture latérale de portion de canal de fluide (11d) de la portion de canal de fluide côté raccord de fluide (11a) dans un état dans lequel l'élément d'activation (30) est dans la position à distance minimale du raccord de fluide (12), afin de former, à travers la chambre de fluide (24), une communication fluidique entre la portion de canal de fluide côté accouplement (11a) et la portion de canal de fluide côté raccord de fluide (11b).

4. Système d'accouplement (300) selon la revendication 2 ou 3, dans lequel la saillie (22) de l'élément d'étanchéité d'élément d'accouplement (20) dirigée vers l'extérieur par rapport à l'axe longitudinal (L1) comporte une portion de fixation (23) qui est fixée dans le boîtier d'élément d'accouplement (10).

5. Système d'accouplement (300) selon la revendication 4, dans lequel le boîtier d'élément d'accouplement (10) comprend une portion de boîtier côté accouplement (10a) et une portion de boîtier côté raccord de fluide (10b) le long de l'axe longitudinal (L1), entre lesquelles la portion de fixation (23) de l'élément d'étanchéité d'élément d'accouplement (20) est retenue.

6. Système d'accouplement (300) selon l'une des revendications 1 à 5, dans lequel l'élément d'activation (30) est formé de façon annulaire, dans lequel en particulier l'anneau de l'élément d'activation (30) vient en prise avec l'extrémité de l'élément d'étanchéité d'élément d'accouplement (20) dirigée vers le raccord de fluide (12).

7. Système d'accouplement (300) selon l'une des revendications 1 à 6, dans lequel le boîtier d'élément d'accouplement (10) comporte, sur le côté d'accouplement (13), au moins une structure de retenue (14), en particulier une structure de retenue (14) faisant saillie axialement par rapport à l'axe longitudinal (L1) au-delà de l'élément d'étanchéité d'élément d'accouplement (20) du côté d'accouplement (13), par l'intermédiaire de laquelle un élément d'accouplement complémentaire (200) peut être retenu dans un état connecté.

8. Système d'accouplement (300) selon la revendication 7, dans lequel la structure de retenue (14) est formée par l'intermédiaire d'au moins deux bras de retenue avec des crochets à encliquetage ou en forme annulaire.

9. Système d'accouplement (300) selon l'une des revendications précédentes, dans lequel une extrémité (43) du boîtier d'élément d'accouplement complémentaire (40) côté accouplement complémentaire peut venire en prise avec le au moins un élément d'activation (30) de l'élément d'accouplement (100) et peut être déplacée conjointement avec celui-ci en direction du raccord de fluide (12) de l'élément d'accouplement (100) dans le boîtier d'élément d'accouplement (10).

10. Système d'accouplement (300) selon la revendication 9, dans lequel l'extrémité (43) du boîtier d'élément d'accouplement complémentaire (40) côté accouplement complémentaire est réalisée sous la forme d'une saillie axiale par rapport à l'axe longitudinal d'élément d'accouplement complémentaire (L2) et forme une portion d'épaulement (47) avec une portion de boîtier du boîtier d'élément d'accouplement complémentaire (40) adjacente à celle-ci.

11. Système d'accouplement (300) selon les revendications précédentes, dans lequel le boîtier d'élément d'accouplement complémentaire (40) comporte, sur une surface extérieure s'étendant axialement par rapport à l'axe longitudinal d'élément d'accouplement complémentaire (L2), une structure de retenue d'élément d'accouplement complémentaire (44), par l'intermédiaire de laquelle l'élément d'accouplement complémentaire (200) peut être retenu dans un état connecté à l'élément d'accouplement (100).

12. Système d'accouplement selon l'une des revendications précédentes, dans lequel le boîtier d'élément d'accouplement complémentaire (40) comprend un canal de fluide d'élément d'accouplement complémentaire (45) avec au moins une ouverture de canal de fluide d'élément d'accouplement complémentaire (46) qui s'étend dans le boîtier d'élément d'accouplement complémentaire (40) en direction de l'axe longitudinal d'élément d'accouplement complémentaire (L2) à partir du raccord de fluide d'élément d'accouplement complémentaire (41) en direction du côté de l'accouplement complémentaire (42), et dans lequel l'élément d'étanchéité d'élément d'accouplement complémentaire (51) rend étanche l'ouverture latérale de canal de fluide d'élément d'accouplement complémentaire (46) dans un état déconnecté, dans lequel l'élément d'étanchéité d'élément d'accouplement complémentaire (51) comporte, entre le joint et l'ouverture d'élément d'étanchéité d'élément d'accouplement complémentaire (51), une chambre de fluide d'élément d'étanchéité d'élément d'accouplement complémentaire (52) qui est déplaçable en direction de l'axe longitudinal d'élément d'accouplement complémentaire (L2) par rapport au canal de fluide d'élément d'accouplement complémentaire (45) afin de former, à l'état connecté, par l'intermédiaire de l'ouverture latérale de canal de fluide d'élément de couplage complémentaire (46), une communication fluidique avec l'intérieur du canal de fluide d'élément de couplage complémentaire (45).
